(19) Europäisches Patentamt
European Patent Office
Office européen des brevets

(11) Numéro de publication: **0 003 269**
**A1**

(12) # DEMANDE DE BREVET EUROPEEN

(21) Numéro de dépôt: 78400220.6

(22) Date de dépôt: 08.12.78

(51) Int. Cl.²: C 07 D 491/22
C 07 D 471/22, A 61 K 31/435
//(C07D491/22, 311/00, 221/00, 221/00, 209/00), (C07D471/22, 221/00, 221/00, 221/00, 209/00)

(30) Priorité: 12.12.77 FR 7737288

(43) Date de publication de la demande: 08.08.79 Bulletin 79/16

(84) Etats contractants désignés: BE CH DE GB IT NL SE

(71) Demandeur: SCIENCE UNION ET Cie SOCIETE FRANCAISE DE RECHERCHE MEDICALE
14 rue du Val d'Or
F-92150 Suresnes(FR)

(72) Inventeur: Offner, Edouard
Les Tilleuls Vattetot sous Beaumont
F-76110 Goderville(FR)

(74) Mandataire: Burtin, Jean-François
SCIENCE UNION ET Cie Service Brevets 22 rue Garnier
F-92200 Neuilly(FR)

(54) **Nouveaux dérivés pentacycliques, leur procédé d'obtention et compositions pharmaceutiques les contenant.**

(57) Dérivés d'acide hétéroyohimbane carboxylique de formule

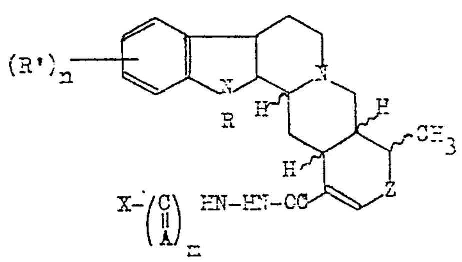

dans laquelle R' représente un radical méthoxy
R représente de l'hydrogène ou un méthyle
Z représente de l'oxygène, un radical imino ou méthylimino
X représente un radical alcoxy inférieur, un groupement amino ou de l'hydrogène.
n représente 0,1 ou 2
m représente 0 et dans ce cas X est de l'hydrogène ou m représente 1 et dans ce cas X est de l'hydrogène, un radical alcoxy ou un radical amino
et A représente de l'oxygène ou un radical imido, ainsi que les N-oxydes, les sels d'ammonium quaternaire et les sels avec un acide minéral organique.

Les composés sont préparés en faisant réagir un dérivé fonctionnel l'acide hétéroyohimbane-16-carboxylique avec une hydrozine de formule $X - \left(\begin{smallmatrix}C\\ \|\\ A\end{smallmatrix}\right)_m - NH - NH_2$

Les composés ont des propriétés pharmacologiques qui les rendent aptes à être utilisés comme principe actif de médicament, notamment comme médicament de l'hypertension et des insuffisances cardiaques ou circulatoirs périphériques.

EP 0 003 269 A1

0003269

La présente invention a pour objet de nouveaux alcaloïdes pentacycliques et leur procédé d'obtention.

Elle a plus particulièrement pour objet de nouveaux hydrazides dérivés d'acides hétéroyohimbane carboxyliques. Elle a spécifiquement pour objet les carbohydrazines pentacycliques répondant à la formule générale I :

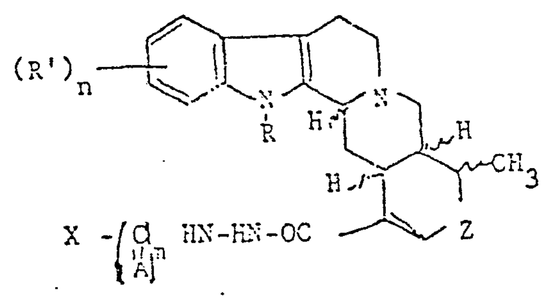

dans laquelle R' représente un radical méthoxy

R représente de l'hydrogène ou un radical méthyle

Z représente de l'oxygène, un radical imino - NH- ou méthylimino $CH_3N$

X représente un radical alcoxy inférieur, un groupement amino ou de l'hydrogène

n représente 0, 1 ou 2

m représente zéro et dans ce cas X est de l'hydrogène, un radical alcoxy ou un radical amino

et A représente de l'oxygène ou un radical imido =NH

L'invention se rapporte également aux sels des composés de formule générale I avec un acide minéral ou organique, de préférence un acide thérapeutiquement compatible. L'un des deux atomes du groupe hydrazino et l'azote N4 sont salifiables et il est donc possible d'obtenir le mono-sel ou le di-sel selon les quantités d'acide mises en réaction.

BAD ORIGINAL

L'invention concerne aussi les N-oxydes des composés de formule générale I obtenus soit à partir d'un N-oxyde naturel soit par synthèse à partir d'une amine tertiaire puis oxydation en N-oxyde. L'invention concerne encore les sels d'ammonium des composés de formule générale I.

Les composés de formule générale I comportent au moins quatre centres d'asymétrie. La configuration des centres en 3, 15, 19 , 20 varie selon la configuration de la matière première-naturelle ou synthétique-utilisée. Les composés racémiques peuvent en outre être dédoublés.

On pourra donc pour obtenir un composé de formule générale I, utiliser notamment comme matière première:

la tétrahydroalstonine (3α,15α,20α H, 19α - $CH_3$)
l'ajmalicine (3α,15α,20β H, 19α - $CH_3$)
l'isoajmalicine (3β,15α,20α H, 19α - $CH_3$)
l'akuammigine (3α,15α,20β H, 19β - $CH_3$)
le N-oxyde d'akuammigine (3α,15α,20β H, 19α - $CH_3$)
la Mayumbine (3α,15α,20α H, 19α - $CH_3$)
l'aricine (3α,15α,20α H, 19α - $CH_3$)
la raumitorine (3α,15α,20β H, 19β - $CH_3$)
la reserpinine (3α,15α,20α H, 19α - $CH_3$)
l'Iso reserpinine (3β,15α,20α H,19α -$CH_3$)
la Tetraphylline (3α,15α,20β H, 19α - $CH_3$)
la Reserpiline (3β,15α,20α H, 19α - $CH_3$)
l'Isoreserpiline (3α,15α,20α H, 19α - $CH_3$)
la 19-epi 3-iso Ajmalicine (3β,15α,20β H,19β - $CH_3$)
la 19-epi Ajmalicine (3α,15α,20β H, 19β-$CH_3$)
la dihydrocathenamine (3α,15α,20α H, 19α - $CH_3$)
la Mitrajavine (3β,15α,20β H, 19α - $CH_3$)

On peut également utiliser comme matière première une base quaternaire pseudo-aromatique comme l'Alstonine ou la Serpentine puis réduire par hydrogénation catalytique ou par un borohydrure de métal alcalin en un composé tétrahydrogéné.

On peut en outre utiliser comme matière première de départ un sel d'ammonium quaternaire comme le Chlorométhylate ou l'iodométhylate

de Reserpiline.

On peut encore utiliser comme matière première un N-oxyde d'Hétéroyohimbane de formule générale II. Après réaction avec l'hydrazine de formule générale III, on réduit si nécessaire le N-oxyde en amine tertiaire.

Parmi les composés actuellement préférés, on pourra citer plus particulièrement les hydrazides de l'acide 16, 17-didehydro 19-méthyl 18-oxayohimbane 16- carboxylique et spécifiquement :

- l'hydrazide de l'acide Raubasinique
- le N-méthylhydrazide de l'acide Raubasinique
- le N'-guanylhydrazide de l'acide Raubasinique
- l'hydrazide de l'acide tétrahydroalstonique et son dichlorhydrate
- l'hydrazide de l'acide 10,11-diméthoxy tétrahydroalstonique
- l'hydrazide de l'acide 9-methoxy 3-iso Raubasinique
- le N'méthyl hydrazide de l'acide 10-méthoxy Raubasinique
- le N'-ethoxycarbonylhydrazide de l'acide tétrahydroalstonique et ses chlorhydrates
- le N'-ethoxy carbonyl hydrazide de l'acide 11-méthoxy-raubasinique et ses chlorhydrates
- le N'-ethoxycarbonylhydrazide de l'acide 10,11-diméthoxy tétrahydroalstonique et ses chlorhydrates.

Les composés de formule générale I ainsi que leurs sels d'addition possèdent des propriétés pharmacologiques intéressantes. Ils sont doués de propriétés hypotensives, tout en améliorant les performances cardiaques. Ils augmentent le débit cardiaque, le débit d'injection systolique et les index cardiaque et systolique. En outre ils diminuent la résistance périphérique totale et la résistance élastique des artères.

Par ailleurs ils ne manifestent qu'une action bradycardisante modérée sans dépression du myocarde et une action neuro-sédative extrêmement atténuée.

C'est pourquoi, ils trouvent un emploi en thérapeutique notamment comme médicament de l'hypertension et des insuffisances cardiaques ou circulatoires périphériques.

On les emploie en vue de la thérapeutique humaine sous forme de compositions pharmaceutiques renfermant à titre de principe actif au moins un composé de formule générale I ou un de des sels en mélange avec un excipient inerte non-toxique, pharmaceutiquement compatible.

Parmi les compositions pharmaceutiques utilisables, on citera plus particulièrement les formes administrables par voie digestive comme par exemple les comprimés enrobés, les dragées, les capsules, les gélules, les comprimés à effet retard, les gouttes, les solutions ou suspensions buvables ou les cachets; les formes administrables par voie parentérale comme par exemple les solutés ou suspensions injectables répartis en ampoules, en flacons multidoses ou en seringues auto-injectables; les formes administrables par voie perlinguale comme les comprimés perlinguaux; les formes pharmaceutiques administrables par voie percutanée comme les solution dans un solvant polaire pour usage percutané; les formes pharmaceutiques administrables par voie rectale comme par exemple les suppositoires .

La posologie utile peut varier largement en fonction de la voie d'administration, de l'indication thérapeutique, de l'âge et du poids du sujet malade.

Elle pourra varier de 5 à 200 mg par prise d'essai chez l'adulte et de 10 à 400 mg par jour, notamment par voie buccale ou parentérale.

L'invention concerne également un procédé d'obtention des composés de formule générale I

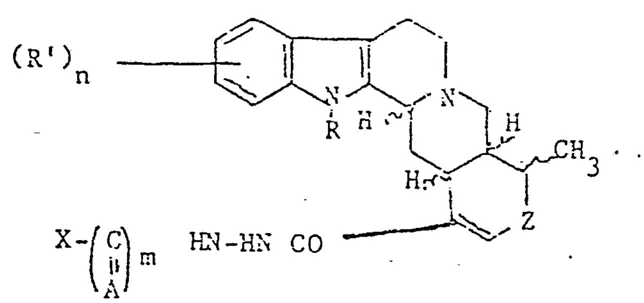

(I)

BAD ORIGINAL

dans laquelle les substituants R', R , A , X , m, Z, et n sont définis comme précedemment

caractérisé en ce que l'on fait réagir un dérivé fonctionnel d'acide hétéroyohimbane 16-carboxylique de formule générale II

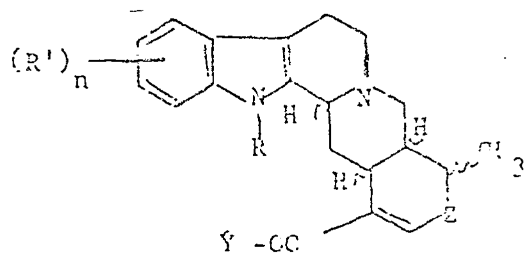

(II)

dans laquelle Y est un halogène, un alcoyle inférieur , un reste d'anhydride mixte ou symétrique ou un reste azidure/ ou un N-oxyde ou un sel d'ammonium quaternaire de celui-ci, avec une hydrazine de formule générale III

$$X - \left(\begin{matrix} C \\ \| \\ A \end{matrix}\right)_m \text{———} NH - NH_2 \qquad (III)$$

dans laquelle X, A et m sont définis comme précédemment

pour former l'hydrazide correspondant que l'on réduit par hydrogénation catalytique ou par un hydrure mixte de métal alcalin lorsque l'atome d'azote en 4 est sous forme de N-oxyde ou de sel d'ammonium quaternaire.

Les composés de formule générale I peuvent être salifiés par addition d'un acide minéral ou organique comme par exemple l'acide chlorhydrique, bromhydrique, l'acide phosphorique, l'acide sulfurique ou l'acide nitrique; l'acide formique, l'acide acétique, l'acide n-di propyl acétique, l'acide tartrique , l'acide citrique, l'acide maléique, l'acide nipecotique, l'acide glucose-1 phosphorique, l'acide méthane sulfonique, l'acide éthane sulfonique , l'acide iséthionique ou l'acide benzène sulfonique.

Les composés de formule générale I peuvent être transformés en leur N-oxyde par action de l'eau oxygénée ou d'un hydroperoxyde de N-

BAD ORIGINAL

oxyde comme par exemple l'hydroperoxyde de N-oxyde de triethylamine ou l'hydroperoxyde de N-oxyde de pyridine.

Selon les modalités actuellement préférées , le procédé selon l'invention peut également être défini comme suit :

1) le dérivé fonctionnel de l'acide hétérohimbène 16-carboxylique est un chlorure d'acide, ou l'anhydride mixte formé par réaction avec un di alcoyl ou un dicyclo carbodi-imide, ou un ester méthylique ou un azidure,

2) l'hydrazine de formule générale III est l'hydrazine, une N-alcoyl hydrazine ou une aminoguanidine,

3) la condensation du dérivé fonctionnel de l'acide de formule II avec l'hydrazine de formule générale III est effectuée dans un solvant à point d'ébullition élevé, compris entre 100 et 200° comme le n-pentanol, l'alcool isobutylique ou le tarbutanol,

4) la condensation du dérivé fonctionnel de l'acide de formule II avec l'hydrazine de formule générale III est effectuée en présence d'un activateur ou d'un catalyseur,

5) les hydrazides sont séparés par salification à l'aide d'un acide minéral;

6) la carbonation de l'hydrazide de formule générale I est effectuée par condensation avec un halogénoformiate d'alcoyle inférieur pour former un carbazate.

Les composés de départ sont décrits dans la littérature. En particulier les hétéroyohimbanes de départ peuvent être soit des produits naturels soit des composés de synthèse. On peut utiliser des composés racémiques ou des composés optiquement actifs. En utilisant une matière première racémique, il est possible d'effectuer ultérieurement un dédoublement en ses isomères optiques par salification à l'aide d'un acide organique optiquement actif.

Les exemples suivants sont destinés à illustrer l'invention. Ils ne la limitent en aucune façon.

EXEMPLE 1

Hydrazide de l'acide tétrahydroalstonique

On met en contact 20g de tétrahydroalstonine, 50ml d'hydrate d'hydrazine et 300ml de n-pentanol et on porte le mélange au reflux en présence de quelques gouttes d'acide acétique pendant 36 heures. On laisse ensuite refroidir et on dilue à l'eau le mélange réactionnel. On élimine le pentanol par distillation sous pression normale de l'azeotrope eau-pentanol. L'hydrazide précipite et on le sépare du milieu aqueux par refroidissement puis filtration. On lave l'insoluble à l'eau et sèche à poids constant sous vide.

On recueille ainsi 14g3 d'hydrazide de l'acide tétrahydroalstonique soit un rendement de 71,5%.

Par chromatographie en couche mince (support silice GF 254 et solvant chloroforme-méthanol) le produit apparait homogène (RF = 0,34)

Pour l'analyse le produit est recristallisé d'un mélange pyridine-eau, on obtient ainsi l'hydrazide de l'acide tétrahydroalstonique pur avec un rendement de recristallisation de 67 %.

Point de fusion de la base recristallisée 225°

<u>Analyse</u> $C_{20} H_{24} N_4 O_2$ = 352,44

| | C | N% |
|---|---|---|
| Calculé | 68,16 | 15,90% |
| Trouvé | 67,48 | 15,74% |

EXEMPLE II

Dichlorhydrate de l'hydrazide de l'acide tétrahydroalstonique.

On met 10g de l'hydrazide de l'acide tétrahydroalstonique obtenu à l'exemple I en suspension dans 90 ml d'éthanol et on dissout le tout par chauffage au reflux . On fait alors barboter un courant d'acide chlorhydrique pendant une heure sous agitation mécanique. On observe une mise en solution puis une précipitation . On arrête le barbotage, on ajoute un volume égal de benzène puis chauffe 3 mn au reflux. On laisse ensuite refroidir une heure en glacière, sépare le dichlorhydrate qui a précipité, par filtration et le sèche sous vide à poids constant.

On obtient le dichlorhydrate de l'hydrazide de l'acide tétrahydroalstonique avec un rendement de 76%.

BAD ORIGINAL

Dosage du chlore

| | |
|---|---|
| Théorie | 16,67 % |
| Trouvé | 16,67 - 16,72% |

EXEMPLE III

N- (ethoxycarbonyl) hydrazide de l'acide tétrahydroalstonique.

On met en suspension à température ordinaire 28 g 16 d'hydrazide d'acide tétrahydroalstonique dans 400 ml d'éthanol puis ajoute 10 g 4 de chloroformiate d'éthyle. On maintient sous agitation pendant une heure puis porte au reflux pendant 10 minutes. On laisse ensuite revenir le mélange cristallin à température ambiante et le maintient sous agitation pendant 1 heure.

On laisse refroidir à - 10°C pendant 45 minutes, puis filtre le précipité que l'on rince à l'éthanol glacé puis sèche sous vide.

On obtient ainsi 29g4 de monochlorhydrate de N'-(ethoxy carbonyl ) hydrazide de l'acide tétrahydroalstonique soit un rendement de 79,8%.

Le produit est purifié par conversion en son dichlorhydrate. On met l'hydrazide en suspension dans 10 volumes d'éthanol au reflux pendant que l'on fait barboter un courant d'acide chlorhydrique pendant une heure. Ensuite on glace le mélange à -10°C, on filtre le précipité que l'on rince à l'éthanol glacé est sèche sous vide. Le rendement en dichlorhydrate est de 65%.

Le produit cristallise sous forme de trihydrate.

CCM sur silice $CF_{254}$ : homogène ( RF = 0,66)

Chlore = 13,67 % ( théorie 12,86 pour le trihydrate)

EXEMPLE IV

Hydrazide de l'acide tétrahydroalstonique

Le composé de l'exemple I peut également être obtenu en opérant au reflux dans l'alcool isoamylique pendant 36 heures.

Le rendement est de 77,5%. La recristallisation de la pyridine fournit un produit pur à plus de 99%.

EXEMPLE V

Monochlorhydrate du N'-(ethoxy carbonyl ) hydrazide de l'acide tétrahydroalstonique.

On agite une suspension de 28g16 d'hydrazide de l'acide tétrahydroalstonique et de 10g4 de chloro formiate d'éthyle à température ambiante pendant une heure. On chauffe ensuite pendant 10 minutes au reflux et laisse revenir à température ambiante. On maintient l'agitation pendant une heure puis laisse au repos pendant une nuit. On filtre le précipité que l'on rince à l'éthanol. On sèche ensuite sous vide.

Les spectres IR et RMN sont compatibles avec la structure

Titre en chlore par argentimétrie : 96%

Perte de poids par chauffage : 4,7%

EXEMPLE VI

Hydrazide de l'acide Raubasinique

En opérant comme à l'exemple I au départ de l'hydrazine et de 50 g d'Ajmalicine , on obtient 26g4 d'hydrazide de l'acide Raubasinique brut (Rdt = 53%)

Par addition d'acide Chlorhydrique dans une solution d'hydrazide dans un mélange acétone-éthanol, le dichlorhydrate cristallise (Rendement 18%)

Les sels de l'hydrazide de l'acide Raubasinique avec l'acide benzoique, l'acide nicotinique, l'acide salicylique , l'acide acétylsalicylique, l'acide Naphtoique ou l'acide p.Toluènesulfonique sont très solubles dans les solvants organiques.

L'hydrazide de l'acide Raubasinique est très soluble dans le méthanol et le n- butanol. Il est soluble dans un mélange pyridine-eau. Il est peu soluble dans les autres solvants.

On peut également isoler l'hydrazide de l'acide Raubasinique par formation du phosphate en extrayant la phase pentanolique à l'acide phosphorique N. On concentre la solution phosphorique à faible volume. Le phosphate précipite par addition de 15 volumes d'ethanol.

Au départ de 80 g d'Ajmalicine on obtient 60g de diphosphate d'hydrazide de l'acide Raubasinique soit un rendement de 56%.

Titre en phosphore 7,3% (théorie 6,9%)

EXEMPLE VII

N'-(ethoxycarbonyl) hydrazide de l'acide Raubasinique.

On met en suspension 18g du diphosphate obtenu à l'exemple VI dans l'éthanol et on ajoute 1,2 équivalent de chloroformiate d'éthyle. On porte 15 minutes au reflux puis laisse refroidir la solution à température ordinaire. On précipite la Carbazate par addition d'ether. On obtient ainsi 8g6 de N'-(ethoxycarbonyl) hydrazide de l'acide Raubasinique.

EXEMPLE VIII

En opérant comme à l'exemple VI au départ de 50g de tetraphylline, on obtient 21g d'hydrazide de l'acide 10-methoxy raubasinique soit un rendement de 42%.

L'hydrazide de l'acide 10-methoxy raubasinique fournit un dichlorhydrate très soluble dans les solvants organiques. Le monophosphate de l'hydrazide de l'acide 10-methoxy raubasinique a été obtenu avec un rendement de 50% (teneur en phosphore 7,47% -théorie 6,96%)

EXEMPLE IX

En opérant comme à l'exemple VI au départ de 72 g de Reserpiline, on obtient 10g3 de diphosphate de l'hydrazide de l'acide 10,11 -dimethoxy - 3 - isotetrahydroalstonique.

Teneur en phosphore 11,23 % (théorie 10,88%)

EXEMPLE X

En opérant comme à l'exemple VII au départ des hydrazides des acides 10-methoxy raubasinique et 10,11-dimethoxy 3-isotetrahydroalstonique, on obtient respectivement :

à partir de 19g2 de phosphate de l'hydrazide de l'acide 10-methoxy raubasinique, 13g3 de N'-(ethoxycarbonyl) hydrazide de l'acide 10-methoxyraubasinique- son Rf en CCM (support silice) est 0,56.

à partir de 2g3 de phosphate de l'hydrazide de l'acide 10,11-dimethoxy 3 - isotétrahydroalstonique , 1g3 de N'-(ethoxy-carbonyl ) hydrazide de l'acide 10,11 -dimethoxy 3- isotétrahydroalstonique.

En CCM (support silice -eluant chloroforme - Methanol) Rf= 0,80.

EXEMPLE XI

N'-methylhydrazide de l'acide 11-methoxy raubasinique.

On dissout 8gl d'acide 11-methoxyraubasinique dans 40ml de chlorure de thionyle dans 20ml de benzène. On refroidit le mélange en dessous de 5° et on maintient à cette température pendant une heure. On chasse l'excés de réactif et l'acide chlorhydrique formé par évaporation à sec sous vide. Le résidu sec est constitué essentiellement par le chlorure de l'acide 11-methoxyraubasinique. On l'utilise tel quel en le reprenant par 25 ml de chlorure de méthylène, et 2 ml de pyridine et on ajoute progressivement une solution de 1g60 de N-methylhydrazine dans 15 ml de chlorure de méthylène. On maintient sous agitation pendant une heure puis lave la solution organique à l'eau jusqu'à neutralité des eaux de lavage. On épuise alors la solution chlorométhylénique avec de l'acide phosphorique N. La solution aqueuse est ensuite saturée d'éther d'où le phosphate du N-methylhydrazide de l'acide 11-methoxy Raubasinique précipite. Pour l'analyse, le phosphate est recristallisé de l'ethanol aqueux.

Teneur en phosphore : 6,85%

Les spectres IR et RMN sont en accord avec la structure.

L'acide 11-methoxy raubasinique a été obtenu selon le procédé décrit dans le brevet belge 764.950.

EXEMPLE XII

Hydrazide de l'acide $N_1$ - methyltétrahydroalstonique.

Au départ de la tétrahydroalstonine, on forme la $N_1$-methyltetrahydroalstonine par action du bromure de méthyle en présence de lessive de soude.

La $N_1$-methyl tetrahydroalstonine fond à 181°.

Analyse $C_{22} H_{27} N_2 O_3$ = 366;46

| | C | H | N% |
|---|---|---|---|
| Calculé | 72.11 | 7.15 | 7.64 |
| Trouvé | 71.87 | 7.11 | 7.63 |

En utilisant le mode opératoire de l'exemple VI au départ de la $N_1$-methyl tetrahydroalstonine, on obtient l'hydrazide de l'acide $N_1$-methyl tetrahydroalstonique.

Point de fusion instantané : environ 220°C.

BAD ORIGINAL

EXEMPLE XIII

Hydrazide de l'acide $N_1$- methyl Raubasinique

En opérant selon le mode opératoire de l'exemple XII, on obtient successivement au départ de la Raubasine:

La $N_1$-methyl Raubasine F = 212.2130

Analyse $C_{22} H_{27} N_2 O_3$ = 366,46

| | C | H | N% |
|---|---|---|---|
| Calculé | 72.11 | 7.15 | 7.64 |
| Trouvé | 71.37 | 7.12 | 7.59 |

Les spectres IR et RMN sont compatibles avec la structure annoncée.

L'hydrazide de l'acide $N_1$-methyl Raubasinique : F = 260° environ.

EXEMPLE XIV

Etude pharmacologique des composés selon l'invention:

a) détermination de la toxicité aigue

La dose léthale moyenne a été determinée pour chacun des composés selon l'invention par administration sous-cutanée à des lots de souris (10) de souche swiss pesant 20g environ. On administre des doses croissantes des produits essayés en solution aqueuse. Les animaux sont gardés en observation pendant 8 jours et les morts s'il y en a sont dénombrés. La dose léthale moyenne a été déterminée graphiquement selon la méthode de Lichtf eld et Wilcoxon. Elle s'echelonne selon les produits entre 1000 et 1500 mg/Kg. Les symptômes constatés sont essentiellement une certaine sédation et une diminution de la motricité des animaux.

b) détermination de l'activité hypotensive

L'activité hypotensive des composés selon l'invention a été déterminée chez des chiens préalablement anesthésiés au Nembutal; la pression carotidienne et le rythme cardiaque sont enregistrés avant injection intraveineuse du produit essayé et pendant une période s'étendant pendant 4 heures après l'injection.

Des doses de 5 et 10mg/Kg entrainent une baisse de 25% de la pression

artérielle moyenne sans affecter le rythme cardiaque. A la dose de 20 mg/Kg la pression artérielle moyenne est abaissée d'environ 30 à 35 mm Hg et le rythme cardiaque est diminué d'environ 30%. La durée de cette dose est d'au moins 4 heures. A cette dose le volume respiratoire est à peine diminué ou au contraire pour certains produits même augmenté (50% environ pendant plus d'une heure ).

L'activité hypotensive a été également étudiée sur des chiens rendus hypertendus par néphrosclérose. A la dose de 10 mg/Kg par voie intraveineuse, on constate une augmentation fugace de la pression artérielle suivie d'une baisse importante durant plus de 4 heures. Le rythme cardiaque est également augmenté.

Les modalités d'action des composés selon l'invention ont été en outre étudiés en présence des principaux médiateurs chimiques ( Adrénaline, Sérotonine, Histamine, DMPP). La plupart de ces médiateurs ont leur action sur la pression artérielle du chien partiellement ou totalement inhibée par injection des produits selon l'invention.

c) recherche d'un effet neurologique

Chez la souris, une dose de 100 mg/Kg par voie intraperitonéale entraine une légère ptose des paupières. A la dose de 200 mg/Kg on constate une réduction légère du tonus musculaire et des reflexes. La démarche est légèrement titubante. Une dose de 500 mg/Kg entraine des convulsions. La motricité est diminuée. Chez le rat, à la dose de 500 mg/Kg , on constate l'apparition de tremblements convulsifs, la motricité et la respiration sont un peu diminuées. Il apparait du ptosis.

BAD ORIGINAL

## REVENDICATIONS DE BREVET.-

L'invention a pour objet

1) A titre de composés industriels nouveaux les carbohydrazines pentacycliques de formule générale I

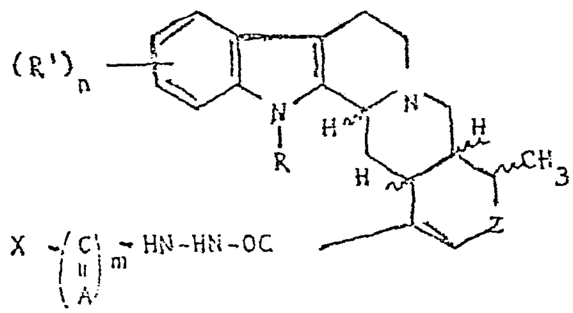

dans laquelle R' représente un radical méthoxy

R représente de l'hydrogène ou un radical methyle

Z représente de l'oxygène , un radical imino -NH- ou méthylimino $CH_3N$

X représente un radical alcoxy inférieur , un groupement amino ou de l'hydrogène

n représente 0, 1 ou 2

m représente zero et dans ce cas X est de l'hydrogène ou m représente 1 et dans ce cas X est de l'hydrogène, un radical alcoxy ou un radical amino

et A représente de l'oxygène ou un radical imido = N-H

2) Les sels des composés de formule générale I selon la revendication 1 avec un acide minéral ou organique, de préférence un acide thérapeutiquement compatible.

3) Les N-oxydes des composés de formule générale I selon la revendication 1.

4) Les hydrazides des acides 16 , 17 didehydro 19-méthyl 18-oxayohimbane 16-carboxylique selon la revendication 1.

5) Un composé selon l'une des reveneications 1 à 4 choisi dans le groupe constitué par

- l'hydrazide de l'acide raubasinique
- le N-méthylhydrazide de l'acide raubasinique
- le N-guanylhydrazide de l'acide raubasinique
- l'hydrazide de l'acide tetrahydroalstonique et son dichlorhydrate
- l'hydrazide de l'acide 10,11 -dimethoxy tetrahydroalstonique
- l'hydrazide de l'acide 11-methoxy raubasinique
- l'hydrazide de l'acide 9-methoxy 3-iso raubasinique
- le N'-methylhydrazide de l'acide 10-methoxy raubasinique
- le N'-ethoxycarbonylhydrazide de l'acide tetrahydroalstonique et ses chlorhydrates
- le N'-ethoxycarbonyl hydrazide de l'acide 11-methoxy raubasinique et ses chlorhydrates

et le N'-ethoxycarbonylhydrazide de l'acide 10, 11-dimethoxy tetrahydroalstonique et ses chlorhydrates.

6) L'application à la thérapeutique des composés selon l'une des revendications 1 à 5, notamment comme médicament hypotensif.

7) Les compositions pharmaceutiques renfermant comme principe actif au moins un composé selon l'une des revendications 1 à 5 en mélange avec un excipient inerte non-toxique pharmaceutiquement acceptable.

8) Les compositions pharmaceutiques selon la revendication 7 dans lesquelles l'excipient inerte est adapté pour l'usage par voie buccale, parentérale, sublinguale, percutanée ou rectale.

9) Les compositions pharmaceutiques selon l'une des revendications 7 et 8 dans lesquelles la teneur en principe actif s'échelonne entre 5 et 200 mg par prise unitaire.

10) Un procédé d'obtention des composés de formule générale I

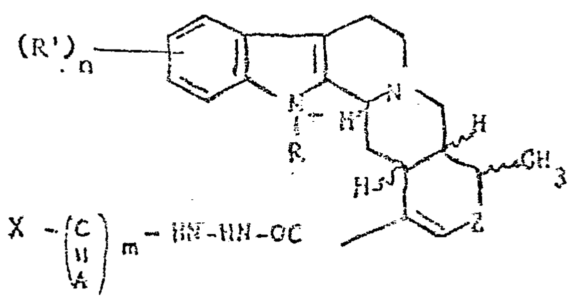

(I)

dans laquelle la définition des substituants R, R', Z,X,A , m et n demeure la même que celle fournie à la revendication 1

caractérisé en ce que l'on fait agir un dérivé fonctionnel d'acide hétéroyohimbane 16-carboxylique de formule générale II

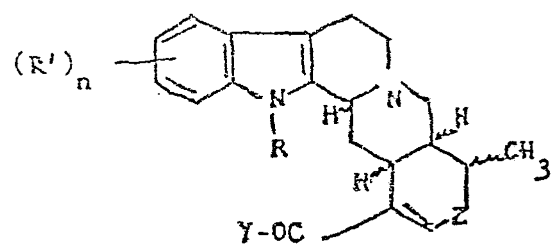

(II)

dans laquelle la définition des substituants R, R', Z demeure inchangée,

et Y est un halogène, un alcoyle inférieur, un reste d'anhydride mixte ou symétrique, ou un reste azidure,

ou un N-oxyde ou un sel d'ammonium quaternaire de celui-ci avec une hydrazine de formule générale III

$$X - \left(\begin{matrix}C\\ \| \\ A\end{matrix}\right)_m - NH - NH_2 \qquad (III)$$

dans laquelle la définition des substituants X, A et m demeure celle fournie à la revendication 1

pour former l'hydrazide correspondant que l'on réduit, si nécessaire, par hydrogénation catalytique ou par un hydrure mixte de métal alcalin, et/ou salifie par addition d'un acide minéral ou organique.

11) Un procédé selon la revendication 10 dans lequel l'hydrazide de formule générale I

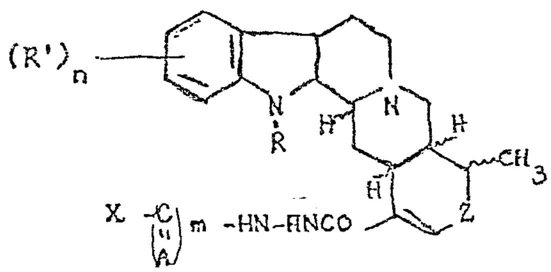

I

dans laquelle m et égal à zero

et X est de l'hydrogène

est soumis à une carbonatation par un halogénoformiate d'alcoyle inférieur pour former le carbazate correspondant de formule générale IV

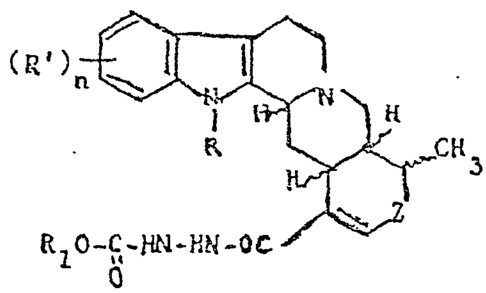

(IV)

dans laquelle $R_1$ est un radical alcoyle inférieur et les substituants R,R', n et Z sont définis comme à la revendicaition 1.

que l'on peut salifier, si désiré, par addition d'un acide minéral ou organique.

BAD ORIGINAL

Office européen des brevets

**RAPPORT DE RECHERCHE EUROPEENNE**

0003269

Numero de la demande

EP 78 40 0220

## DOCUMENTS CONSIDERES COMME PERTINENTS

| Catégorie | Citation du document avec indication, en cas de besoin, des parties pertinentes | Revendication concernée |
|---|---|---|
| P | FR - A - 2 383 949 (OMNIUM CHIMIQUE S.A.)<br>* Revendications *<br>-- | 1 |
| E | DE - A - 2 808 262 (OMNIUM CHIMIQUE S.A.)<br>* En entier *<br>& BE - A - 864 248<br>---- | 10 |

**CLASSEMENT DE LA DEMANDE (Int. Cl.²)**

C 07 D 491/22
471/22
A 61 K 31/435
(C 07 D 491/22, 311/00, 221/00, 221/00, 209/00)
(C 07 D 471/22, 221/00, 221/00, 221/00, 209/00)

**DOMAINES TECHNIQUES RECHERCHES (Int. Cl.²)**

C 07 D 491/22
471/22

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

☒ Le présent rapport de recherche a été établi pour toutes les revendications

| Lieu de la recherche | Date d'achèvement de la recherche | Examinateur |
|---|---|---|
| La Haye | 09-03-1979 | VAN BIJLEN |

OEB Form 1503.1 06.78